# EUROPEAN PATENT APPLICATION

(11) **EP 1 894 548 A1**
(43) Date of publication of application: **05.03.2008**
(21) Application number: 07253430.8
(22) Date of filing: 30.08.2007
(51) Int. Cl.: A61F 7/00

(54) **Heat pad**

(30) Priority: 02.09.2006 GB 0617355
(71) Applicant: Tenacta Group S.p.A., 24052 Azzano S. Paolo (BG) (IT)
(72) Inventor: Milani, Giancarlo, 24030 Mozzo (BG) (IT); Morgandi, Arturo, 24129 Bergamo (IT)
(74) Representative: Bailey, Richard Alan

(57) **Abstract**

A heat pad comprises a heat storage medium (14, 16), and an electric heating element (10) associated with the heat storage medium (14, 16) such that, when energised, the electric heating element (10) heats the heat storage medium (14, 16).

## Description

This invention relates to a heat pad, and in particular to an electrically operated heat pad.

Heat pads are available in a range of forms to provide heat to a specific area, for example a part of the human body. One simple form of heat pad is a hot water bottle or other container within which a hot liquid can be contained. Such devices have been widely available for many years. More recently a range of sealed, liquid or gel filled heat pads have become available in which the pads are designed to be heated before use using a microwave or regular ventilated oven. Other forms of heat pad include an electric heating element to provide heat to the user.

Another known arrangement uses an electrical heating element to heat a ceramic disc. Heat is gradually dissipated by the ceramic disc, even after disconnection of the electrical supply.

Although all of these forms of heat pad can function adequately, they all suffer from disadvantages. For example, filling a hot water bottle or the like with hot water can be inconvenient and carries the risk of the user scalding himself. Oven heated pads have the risk that the pad may burst leading to the heated fluid escaping with the associated risk of scalding/burning. Further, if overheated, the user could burn himself whilst trying to remove the pad from the oven. Electrically heated devices have the disadvantage that they can only be used close to an electrical supply socket, and the provision of a power cable connecting the pad to the supply socket can be inconvenient or form a trip hazard. If the pad is disconnected from the power supply, for example to relieve the inconvenience of having to have the power cable, to reduce the risk of tripping or to allow the use of the heated pad at a location remote from the supply socket, then the heat pad will very rapidly cool down and so will no longer be able to perform its intended purpose. The arrangement using a ceramic disc can be uncomfortable to use as the ceramic disc is located within a rigid housing.

It is an object of the invention to provide a heat pad in which these disadvantages are overcome or are of reduced effect.

According to the invention there is provided a heat pad comprising a heat storage medium, and an electric heating element associated with the heat storage medium such that, when energised, the electric heating element heats the heat storage medium.

Such an arrangement is advantageous in that, once the heat storage medium has been heated, the electric heating element can be de-energised and the heat stored in the heat storage medium will gradually be dissipated from the heat storage medium to heat the desired area, for example a part of the human body. The use of an electric heating element to heat the heat storage medium enables heating of the heat storage medium to be carried out in a simple convenient and safe manner. Further, as heat is stored and subsequently dissipated from the heat storage medium, the heat pad can be used to provide heat at locations remote from the electrical supply.

The heat storage medium is preferably flexible. As a result, comfort of the user is enhanced.

The electric heating element is conveniently connected to a control circuit to control operation thereof. The control circuit may be arranged to switch off the electric heating element after a predetermined time and/or once a predetermined temperature has been attained. The control circuit may include one or more thermostats to determine when the desired temperature has been attained. Alternatively, this may be determined by, for example, monitoring the resistance of the electric heating element.

To avoid overheating of the heat storage medium, as mentioned above, the control circuit may be arranged to switch off the heating element after a predetermined period of time, for example after 20 minutes of heating, or after the heating element has been held at or above a predetermined temperature for a period of time. With such an arrangement there is still the risk that overheating may occur if, for example, the heat storage medium is already warm or hot at the commencement of the heating cycle. Conveniently, the control circuit is arranged to sense the temperature of the heating element at the commencement of the heating cycle, and to reduce the maximum heating time and/or reduce the heating power in the event that the initial temperature exceeds a predetermined level.

The electric heating element is conveniently connectable to a connecting cable by means of a detachable connector. It will be appreciated, therefore, that the inconvenience of a power supply cable which is permanently connected to the heat pad can be avoided.

The heat storage medium may comprise a layer of a heat storage material located adjacent the electric heating element. The heat storage medium may further comprise a second layer of heat storage material, the electric heating element being conveniently located between the layers of heat storage material.

The heat storage material may take a number of forms. For example it could comprise a water based gel material, or plastics material beads. However, preferably the heat storage material comprises a phase change material such as the Rubitherm GR series of materials, in which a phase change component is encapsulated in or associated with a granular material. Alternative forms of material include the Rubitherm PK and PX series of materials.

The heat storage medium and electric heating element are conveniently encased within a cover. For example a wipe-clean flexible plastics cover may be provided. A removable fabric outer cover may also be provided, if desired.

The invention will further be described, by way of example, with reference to the accompanying drawings, in which:
Figure 1 is an illustration of a heat pad in accordance with an embodiment of the invention;
Figure 2 is a diagrammatic sectional view of part of the heat pad of Figure 1; and
Figure 3 illustrates an alternative embodiment.

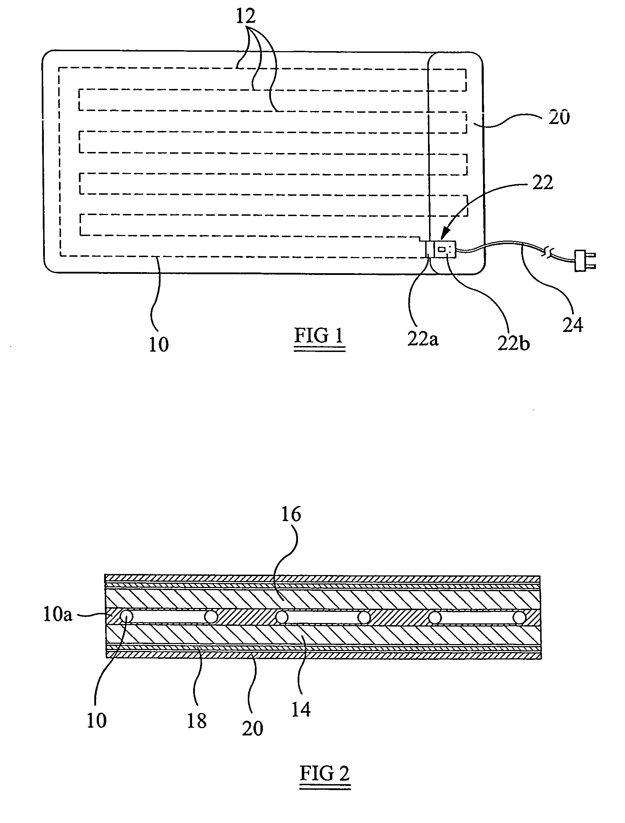

The heat pad illustrated in Figures 1 and 2 of the accompanying drawings comprises an elongate electrical heating element 10 which is laid out in a series of generally parallel runs 12, for example as illustrated by the dashed lines in Figure 1. The heating element 10 is sandwiched between a pair of layers 14, 16 of a heat storage material, the layers 14, 16 together forming a heat storage medium. The assembly of the heating element 10 and layers 14, 16 is located within a permanent cover or casing 18 which itself is located within a fabric material cover 20. The fabric material cover 20 is preferably removable for washing purposes. By way of example, the cover 20 may be adapted to be secured in position using a velcro-like fastening, but it will be appreciated that other arrangements are possible.

The heating element 10 conveniently comprises a conventional helically wound, PVC insulated element located within or upon a thin fabric or foam carrier sheet 10a which may be adhered to the layers 14,16. However, other forms of heating element may be used. For example the heating element could be in the form of a pair of concentrically arranged conductors, conductive strips, a p.t.c. type heating fabric or a range of other forms.

As illustrated in Figure 1, both ends of the electrical heating element 10 are connected to one part 22a of a connector 22, the other part 22b of which is connected to a power supply cable 24 to allow the detachable coupling of the power supply cable 24 to the remainder of the heat pad. Where, for example, the heating element 10 is of the coaxial type having concentrically arranged conductors, or of a similar form, the conductors may be connected to one another at one end of the element, in which case only the other end of the element may be connected to the connector 22.

In addition to connecting the cable 24 to the heat pad, the connector 22 may also house the components of a control circuit for controlling the operation of the heat pad. Alternatively, these components, if provided, may be located within a separate housing provided elsewhere, for example part-way along the power supply cable 24, if desired.

The heat storage material used in the layers 14,16 is conveniently of a phase change material such as one of the materials sold under the Rubitherm GR trade name. This material has the advantage that it has a high thermal energy storage capacity and that heat storage and release takes place at a fairly constant temperature. It is of granular form, the phase change component of the material being secured to or within the granules, and it has been found that the encapsulated nature of the phase change component tends to result in minimal loss of the component over time, in use. The overall material is consequently relatively convenient to use in production and is of good safety. Although preferably one of the Rubitherm GR materials is the material used in the layers 14,16, it will be appreciated that a number of other materials could be used.

In use of the heat pad, the heat pad is connected to a suitable electrical supply socket and the electrical heating element 10 energised so as to heat the layers 14,16 of heat storage material. After a period of time, the electrical supply can be switched off in order to de-energise the heating element 10 and the power supply cable 14 can be disconnected from the heat pad. The heat stored in the layers 14,16 of heat storage material will gradually be dissipated therefrom. The heat pad can thus be used to heat localised areas in the same way as can be achieved with conventional devices.

Where a phase change material such as Rubitherm GR is used as the heat storage material, the heat storage component of the material is typically in the form of a solid at room temperatures. Although the component is solid at room temperatures, the granular nature of the material encapsulating the component ensures that overall the material is still flexible and so the heat pad is comfortable to use. When heated to its melting temperature, the phase change component of the material changes to its liquid phase, absorbing a very large amount of heat whilst remaining at a substantially constant temperature. Overall, the material is still of granular form and so remains comfortable to use. The high latent storage capacity of this material means that upon disconnection of the electrical supply, a large amount of heat can be released, again at a substantially constant temperature at approximately the melting point of the component, until the component has returned from its liquid phase back to its solid phase. Once back in its solid form, the component will cool back down to room temperature.

Apart from having a high thermal energy storage capacity, other advantages of using a phase change material such as Rubitherm GR as the heat storage material are that the material is non-toxic, is 100% recyclable and has long useful working life. The material is available in a range of forms with different melting points, and the material used can thus be selected to suit the application in which it is to be used. By way of example, melting points of between 40°C and 100°C are available. The material is not electrically conductive, thus the heat pad is of enhanced safety as, even in the unlikely event of the material coming into contact with the heating element, for example due to a breakage in the insulating material of the element, the risk of an electric shock is very low.

If desired, the control circuitry used in controlling operation of the heat pad may be arranged to monitor the resistance provided by the heating element 10. Such an arrangement enables the safe use of a heating element with a higher power rating, for example approximately double the usual rating, and hence a reduced required heating time can be achieved in a safe manner. This can be achieved electronically using, for example, a precision microprocessor to control the electrical supply to the heating element 10 such that the final temperature of the heating element 10 is, for example, just above the melting point of the heat storage material.

In one envisaged arrangement, the control circuitry includes a microprocessor programmed to avoid overheating of the heat storage material even if the heat pad is left switched on for an extended period of time or if a new heating cycle is commenced before the heat storage material has cooled to room temperature. For example, the microprocessor may be programmed to switch off the electrical supply to the heating element 10 after a predetermined period of time from the commencement of the heating cycle, for example after a period of 20 minutes. Alternatively, it may be arranged to monitor the temperature of the heating element 10 and to switch off the electrical supply once the temperature has exceeded a predetermined level for a predetermined period of time. To guard against overheating due to commencing a heating cycle when the heat storage material is already warm or hot, conveniently the temperature of the element 10 is sensed at the commencement of the heating cycle, and the heating cycle modified dependent upon the sensed temperature. For example, if it is sensed that the material is already warm or hot, the duration of the heating cycle may be shortened and/or the heat output of the element reduced. It will be appreciated that the avoidance of overheating is important both for safety reasons and also to avoid damage to or loss or escape of the heat storage material,

If desired, rather than use a microprocessor controlled arrangement, a simple thermostat could be provided and arranged to switch off the electrical supply upon the temperature reaching a desired temperature.

If required, the control circuit as well as controlling heating, may also be arranged to illuminate a lamp or LED, or provide an alternative indication, once the heat pad is ready for use, i.e. once the heat storage material has changed phase completely. Once this point has been reached, the control circuitry could be arranged to maintain the heat pad in this condition until the user disconnects the heat pad from the electrical supply. As a result, the safety of the product is enhanced, and the risk of overheating of the pad can be minimised.

Although in the description hereinbefore, it is envisaged that the electrical supply would be a mains electrical supply, this need not always be the case.

Although the heat pad illustrated and described hereinbefore is of generally rectangular form, it will be appreciated that the invention is not restricted to arrangements of this form, and the heat pad could be designed in such way as to be used with specific parts of the human body, for example it could be designed to be fitted around knee or elbow joints, if desired. Although illustrated as a flat rectangular pad, it could be designed to adopt a flexible three dimensional shape, if desired.

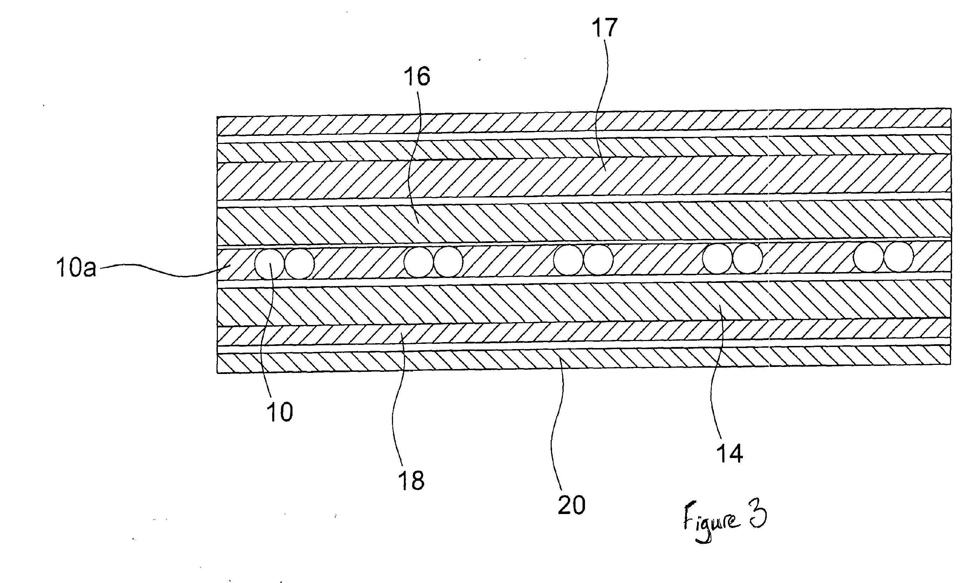

Figure 3 illustrates an arrangement similar to that of Figures 1 and 2 but in which, between the layer 16 of heat storage material and the cover or casing 18 on one side of the heat pad, a layer 17 of a thermally insulating foam material is provided. The provision of the layer 17 serves to insulate one side of the heat pad so that most heat energy transmitted from the heat pad is transmitted from the side thereof without the layer 17. The foam material of the layer 17 is compliant and so comfortable for the user to use. Although the provision of a foam material layer enhances comfort, there may be applications in which other materials could be used to form the insulating material layer 17. By thermally insulating one side of the heat pad, the loss of heat energy from the heat pad during the heating cycle may be reduced so it may permit the duration of the heating cycle to be reduced.

A range of other modifications or alterations will be apparent to the man skilled in the art and it will be appreciated that the invention covers such modifications or alterations and is not restricted to the specific arrangements described and illustrated herein.

## Claims

1. A heat pad comprising a heat storage medium, and an electric heating element associated with the heat storage medium such that, when energised, the electric heating element heats the heat storage medium.

2. A heat pad according to Claim 1, wherein the heat storage medium is flexible.

3. A heat pad according to Claim 1 or Claim 2, wherein the electric heating element is connected to a control circuit to control operation thereof.

4. A heat pad according to Claim 3, wherein the control circuit is arranged to switch off the electric heating element after a predetermined time and/or once a predetermined temperature has been attained.

5. A heat pad according to Claim 4 wherein the control circuit includes one or more thermostats to determine when the desired temperature has been attained.

6. A heat pad according to Claim 4, wherein the temperature of the heat pad is determined by monitoring the electrical resistance of the electric heating element.

7. A heat pad according to Claim 4, wherein the control circuit is arranged to switch off the heating element after the heating element has been held at or above a predetermined temperature for a period of time.

8. A heat pad according to any of Claims 4 to 7, wherein the control circuit is arranged to sense the temperature of the heating element at the commencement of the heating cycle, and to reduce the maximum heating time and/or reduce the heating power in the event that the initial temperature exceeds a predetermined level.

9. A heat pad according to any of the preceding claims, wherein the electric heating element is connectable to a connecting cable by means of a detachable connector.

10. A heat pad according to any of the preceding claims, wherein the heat storage medium comprises a layer of a heat storage material located adjacent the electric heating element.

11. A heat pad according to Claim 10, further comprises a second layer of heat storage material, the electric heating element being located between the layers of heat storage material.

12. A heat pad according to Claim 10 or Claim 11, wherein the heat storage material comprises a phase change material.

13. A heat pad according to any of the preceding claims, further comprising a layer of a thermally insulating material located to one side of the heating element.
